# EUROPÄISCHE PATENTSCHRIFT

(11) **EP 1 064 002 B1**
(45) Veröffentlichungstag und Bekanntmachung des Hinweises auf die Patenterteilung: **26.09.2001**
(21) Anmeldenummer: 99914511.3
(22) Anmeldetag: 11.03.1999
(51) Int. Cl.: A61K 31/66

(54) **VERWENDUNG VON SPHINGOSIN-1-PHOSPHAT, SPHINGOSIN-1-PHOSPHAT-DERIVATEN UND/ODER DEREN GEMISCHE ZUR BEHANDLUNG VON ENTZÜNDLICHEN HAUTKRANKHEITEN**
USE OF SPHINGOSIN-1-PHOSPHATE, SPHINGOSIN-1-PHOSPHATE DERIVATIVES AND/OR MIXTURES THEREOF FOR THE TREATMENT OF INFLAMMATORY DISEASES OF THE SKIN
UTILISATION DE SPHINGOSINE-1-PHOSPHATE, DE DERIVES DE SPHINGOSINE-1-PHOSPHATE ET/OU DE LEURS MELANGES POUR TRAITER LES MALADIES INFLAMMATOIRES CUTANEES

(30) Priorität: 13.03.1998 DE 19810999
(43) Veröffentlichungstag der Anmeldung: 03.01.2001
(73) Patentinhaber: Dermapharm GmbH, 82026 Grünwald (DE); Schäfer-Korting, Monika, 14195 Berlin (DE); Korting, Hans-Christian, 85764 Oberschleissheim (DE); Kleuser, Burkhard, 14476 Fahrland (DE); Gerber, Rolf, 5643 Sins (CH)
(72) Erfinder: SCHÄFER-KORTING, Monika, D-14195 Berlin (DE); KORTING, Hans-Christian, D-85764 Oberschleissheim (DE); KLEUSER, Burkhard, D-14476 Fahrland (DE); GERBER, Rolf, CH-5643 Sins (CH)
(74) Vertreter: Becker Kurig Straus
(86) Internationale Anmeldenummer: EP9901601
(87) Internationale Veröffentlichungsnummer: WO9947145

(56) Entgegenhaltungen:
- US-A- 5 260 288
- US-A- 5 391 800
- US-A- 5 627 171
- US-A- 5 663 404
- US-A- 5 712 262

## Beschreibung

Die Erfindung betrifft eine neue Verwendung von Sphingosin-1-Phosphat, Sphingosin-1-Phosphat-Derivaten und/oder Gemischen der vorgenannten Substanzen, zur Herstellung eines Arzneimittels zur Behandlung von entzündlichen Hautkrankheiten, d.h. insbesondere von Psoriasis vutgaris, Acne vulgaris oder von Hauterkrankungen, die chronische Lichtschädigung der Haut beinhalten, die auch mit Kligman als Dermatoheliosis bezeichnet werden.

Die Erfindung betrifft daher Arzneimittel zur Behandlung der oben genannten entzündlichen Hautkrankheiten, welche Sphingosin-1-Phosphat bzw. Sphingosin-1-Phosphat-Derivate und oder Gemische der vorgenannten Substanzen als Wirkstoff sowie übliche Träger-, Hilfs- und Verdünnungssubstanzen enthalten. Sie betrifft Arzneimittel mit neuartigen Wirkstoffträgern, wie Liposomen und sogenannten solid lipid nanoparticles. Vorzugsweise betrifft die Erfindung topisch applizierbare Mittel zur Behandlung von Psoriasis vulgaris. Acne vulgaris oder Dermatoheliosis.

Die seit langem in der externen Dermatotherapie verabreichten Glukokortikoide sind nach wie vor die wichtigsten entzündungshemmenden Wirkstoffe. Sie nehmen in der lokalen Theraphie den breitesten Raum ein. Darüber hinaus wirken diese Stoffe antiphlogistisch und anti-prurituös sowie antiproliferativ. Erstere Wirkungen spielen bei der Hauptindikation der Glukokortikoide, nämlich akuten entzündlichen Reaktionen der Haut, die wesentliche Rolle. Der letztere Effekt ist vor allem bei der Psoriasis wichtig.
Glukokortikoide beeinflussen die Proliferation von Keratinozyten und Fibroblasten im Sinne einer Hemmung. Dies kann für entzündliche Hautkrankheiten mit hyperproliferativer Komponente eine erwünschte Wirkung sein. So werden Glukokortikoide auch bei der Schuppenflechte eingesetzt. Die Wirkung auf die Fibroblasten kann zur Hautverdünnung

Eine wichtige Zielerkrankung stellt somit die Psoriasis vulgaris dar. Sie ist bei hellhäutigen Menschen die häufigste Hauterkrankung mit polygener Vererbung und multifunktionaler Auslösung. Psoriasis vulgaris ist im wesentlichen gekennzeichnet durch überstürzte Epidermis-Bildung (Hyperproliferation), d. h. eine Keratinozyten-Wanderung von der Basalschicht bis zur Hornschicht von ca. 4 Tagen (normalerweise beträgt diese 28 Tage). Weiterhin wurde bei psoriatischer Haut eine deutlich höhere Resorption und zugleich eine verkürzte Ausscheidung von Fremdstoffen im Urin festgestellt und mit der Zerstörung der Barriere- und Reservoir-Funktion der oberen Hautschichten erklärt.

Bei der Acne vulgaris handelt es sich um eine in der Pubertät, selten später auftretende gelegentlich bis zum 30. Lebensjahr anhaltende Hautkrankheit, die durch eine Talgdrüsen-Hyperplasie und eine Verhornungsstörung der Follikel zu deren Verstopfung mit Bildung von Komedomen, den für die Acne vulgaris typischen Effloreszenzen, führt. Bei der Acne vulgaris liegt insbesondere auch eine Störung der Zelldifferenzierung vor. Üblicherweise wird die Akne keratolytisch mit Benzoyiperoxid oder Tretinoin oder antimikrobiell mit Erythromycin, Clindamycin oder Azelainsäure behandelt. Systemisch besteht die Möglichkeit, antimikrobiell mit Tetracyclinen bzw. antiseborrhoisch mit Antiandrogenen oder Östrogenen zu arbeiten bzw. das systemische Retinoid Isotretinoin einzusetzen.

Immer häufiger wird auch eine Hautkrankheit beobachtet, die als eine chronische Lichtschädigung der Haut zu verstehen ist, und die man mit Kligman als Dermatoheliosis bezeichnet. Eine wichtige Erkrankung aus diesem Formenkreis ist eine Vorstufe des Stachelzellkrebses der Haut, die aktinische oder solare Keratose. Hier liegt eine schwerwiegende Differenzierungsstörung der Epidermis vor.

Sphingolipide umfassen eine große Klasse von biologisch bedeutsamen Verbindungen. Derartige Verbindungen sind Bestandteile von Zellmembranen. Eine wesentliche Verbindung der Sphingolipide ist das Sphingosin.

Befinden sich die Sphingolipide in der Zelle, werden sie metabolisiert, um Sphingosin als metabolisches Intermediat zu ergeben. Sphingosin-1 -Phosphat ist als Produkt der Sphingosin-Kinase bekannt (siehe z.B. Stoffel, W., Hoppe-Seyler's Z. Physiol. Chem., 354, 562, 1973).

Sphingolipid-Abbauprodukte wie Ceramid, Sphingosin und Sphingosin-1-Phosphat werden in der Literatur als neue sogenannte "second messenger" diskutiert. Sphingosin und andere Abbauprodukte des Sphingosin-Metabolismus regulieren entweder Apoptose oder mitogene Effekte, was in Abhängigkeit vom Zelltyp und der Natur des Stimulus zu sehen ist.

Apoptose ist ein essentieller physiologischer Prozess, der hilft, Zellzahlen zu regulieren. WO 96/18404 beschreibt in diesem Zusammenhang, daß Sphingosin und N-Methylderivate von Sphingosin Apoptose in bestimmten Zelltypen induzieren.

WO 88/01869 beschreibt in diesem Zusammenhang die Wirkung von Sphingosin als Inhibitor der Proteinkinase C. Eine Inhibierung der Proteinkinase C führt in einer Vielzahl von Zellen zu einer Inhibierung der Proliferation und zur Induktion des programmierten Zelltodes. Dieser Zelltod von z.B. Keratinozyten, in höheren Konzentrationen von Sphingosin, beruht auf einer cytotoxischen Wirkung nach Hemmung der Proteinkinase C.

im Gegensatz zu der zuvor beschriebenen Wirkung besitzt Sphingosin in einer Reihe von Fällen wie beispielsweise in Fibroblasten eine proliferationsfördernde Wirkung. Dieser proliferative Effekt ist stereospezifisch nur durch das D(+)-erythro-Enantiomer induzierbar.
Auf der anderen Seite ist die Hemmung der Proteinkinase C unspezifisch und lässt sich für alle enantiomeren Verbindungen zeigen.

Weiterhin wird diskutiert, daß in Swiss 3T3-Fibroblasten Sphingosin-1-Phosphat die Proliferation fördert. Zahlreiche intrazelluläre Abläufe werden durch Sphingosin-1-Phosphat beeinflußt. So erhöht Sphingosin-1-Phosphat intrazelluläre Ca²⁺-Ionenkonzentration aus intrazellulären Speichern auf einem IP₃-unabhängigen Weg und senkt die intrazelluläre Konzentration von cAMP. Während die Erhöhung des Ca²⁺-Gehaltes auf eine intrazelluläre Wirkung zurückzuführen ist, hat man seit kurzem auch membranständige Rezeptoren für Sphingosin-1-phosphat entdeckt. Dabei ist die inhibitorische Wirkung auf den CAMP Gehalt auf einen Rezeptoreffekt zurückzuführen.

Aus WO 95/21848 sind verschiedene Syntheseverfahren für Sphingosin-1-Phosphat bzw. für verschiedene Sphingosin-1-Phosphat-Derivate bekannt.

WO 93/19760 beschreibt die Auswirkung von Sphingosin-1-Phosphat und seinen Derivaten auf die Zellbeweglichkeit. Ebenfalls wird in WO 93/19760 die Verwendung von Sphingosin-1-Phosphat als Antitumor-Mittel beschrieben.

Es ist daher Aufgabe der Erfindung, neue Wirkstoffe zur Behandlung von entzündlichen Hautkrankheiten und insbesondere von Psoriasis vulgaris, Acne vulgaris oder Dermatoheliosis bereitzustellen. Weiterhin soll ein neues Arzneimittel, das die vorgenannten neuen Wirksubstanzen enthält, bereitgestellt werden. Das Arzneimittel soll insbesondere topisch applizierbar sein, es soll ein Tensid-freier Arzneistoffträger zur Verfügung gestellt werden, der eine Dispersion von Teilchen in einem wäßrigen Medium bilden kann, wodurch die Aufnahme des Wirkstoffes in die Haut begünstigt wird.

Die oben genannte Aufgabe wird durch die neuartige Verwendung von Sphingosin-1-Phosphat, Sphingosin-1-Phosphat-Derivaten der allgemeinen Formel I, 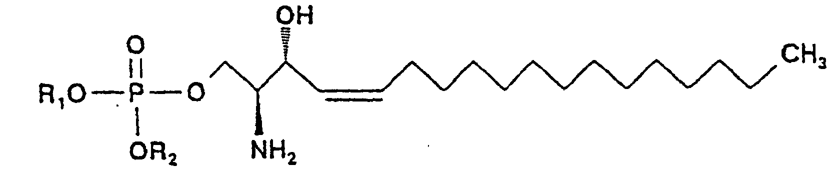 worin R₁ ein Alkylrest mit 1 bis 6 C-Atomen und
R₂ = Wasserstoff oder 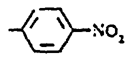 ist, gelöst.

Die oben genannte Aufgabe wird durch die neuartige Verwendung von Sphingosin-1-Phosphat, Sphingosin-1-Phospbat-Derivaten der allgemeinen Formel I, 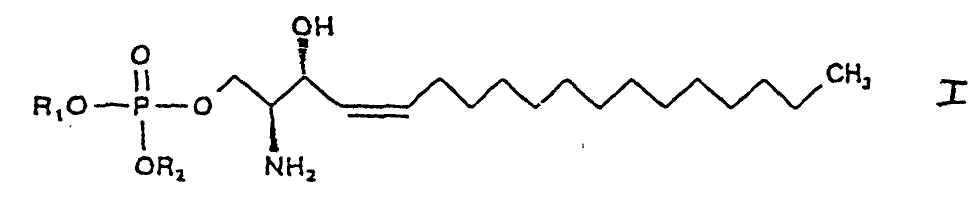 worin R₁ ein Alkylrest mit 1 bis 6 C-Atomen und
R₂ = Wasserstoff oder 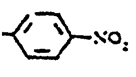 ist, gelöst.

In den Unteransprüchen sind vorteilhafte Ausführungsformen der Erfindung enthalten.

Überraschenderweise wurde nämlich durch die Erfinder festgestellt, daß Sphingosin-1-Phosphat sowie Derivate dieser Substanzen die Proliferation von Keratinozyten hemmen, während sie ihre Differenzierung fördern, und das bei fehlender Proliferation-hemmender Wirkung auf Fibroblasten.

Erfindungsgemäß wurde gezeigt, daß insbesondere Sphingosin-1-Phosphat Einfluß auf das Wachstumsverhalten und die Differenzierung von Keratinozyten und Fibroblasten als den wichtigsten Zellen der Haut hat. Erstmalig konnte gezeigt werden, daß Sphingosin-1-Phosphat die Proliferation von primären Fibroblasten stimuliert, während die Proliferation der Keratinozyten stärk abnimmt. Eine profilerationshemmende Wirkung von Sphingosin-1-phosphat ist bisher in keinem anderen Zelltyp gezeigt worden. Verschiedene Sphingosin-1-Phosphate der oben genannten Formel l zeigten ähnliche Ergebnisse.

Die Ergebnisse zeigen eindeutig, daß der Effekt spezifisch für Sphingosin-1-Phosphat ist, etwas abgeschwächt findet er sich auch bei anderen Sphingosin-Derivaten. Besonders wichtig. ist, daß der anti-proliferative Effekt von Sphingosin-1-Phosphat nicht auf einer cytotoxischen Wirkung beruht, wie sie bei Anti-Tumor-Mitteln vorliegt, und daß das Differenzierungsprofil der Zellen beeinflußt wird. So induziert Sphingosin-1-Phosphat die Differenzierung von Keratinozyten. Daher liegt die Verwendung von Sphingosin-1-Phosphat, Sphingosin-1-Phosphat-Derivaten der oben genannten Formel I in der Behandlung von entzündlichen hyperproliferierenden Hautkrankheiten auch nicht nahe.

Die erfindungsgemäßen Ergebnisse belegen somit, daß die Wirkung von Sphingosin-1-phosphat nicht auf einer Hemmung der Proteinkinase C beruht. Viel mehr verursacht Sphingosin-1-phosphat einen antiproliferativen Effekt, der nicht mit einer erhöhten Zytotoxizität verknüpft ist.

Auch die Differenzierung von Keratinozyten wird durch Sphingosin-1-phosphat gefördert, ein Ergebnis was mit der Stimulierung der Proteinkinase C verbunden ist und konträr zu einer Hemmung der Proteinkinase C steht.

Erfindungsgemäß lässt sich somit nachweisen, daß Sphingosin-1-phosphat zur Behandlung von hyperproliferierenden Hautkrankheiten sehr geeignet ist.

Weiterhin ist zu bemerken, daß eine Aktivierung der Proteinkinase C auch zu einem protektiven Effekt bezüglich der Apoptose führt. Aktivatoren der Proteinkinase C hemmen den programmierten Zelltod. Erfindungsgemäß konnte dabei gezeigt werden, dass dieser protektive Effekt zumindest teilweise auf eine Stimulation der Sphingosin-Kinase durch die Proteinkinase C zurückzuführen ist. Die Sphingosin-Kinase ist das entscheidende Enzym zur Bildung von Sphingosin-1-phosphat. In Analogie besitzt Sphingosin-1-phosphat ebenfalls einen protektiven Effekt bei der Apoptose.

In Keratinozyten ist daher die Proteinkinase C ein zentrales Enzym, welches an der Induktion der Differenzierung beteiligt ist. Eine Stimulation des Enzyms durch Phorbolester ist mit differenzierungsfördernden Einflüssen verbunden. Durch Hemmung der Proteinkinase C wird in Keratinozyten zwar ein antiproliferativer Effekt erreicht. Dieser ist jedoch mit dem Zelltod der Keratinozyten verbunden.

Im Gegensatz dazu besitzt das erfindungsgemäß eingesetzte Sphingosin-1-Phosphat keinen toxischen Effekt in Keratinozyten. Darüber hinaus induziert ein Aktivierung der Proteinkinase C auch ein Stimulierung der Sphingosin-Kinase und führt zu erhöhten Sphingosin-1-phosphat―Spiegeln. Der differenzierungsfördernde und antiproliferative Effekt von Sphingosin-1-phosphat beruht somit in keinem Fall auf einer Inhibierung der Proteinkinase C, wie dies beispielsweise in WO88/01869 für Sphingosin beschrieben wird.

Calcitriol (aktiver Metabolit des Vitamin -D3) sowie Calcipotriol haben sich als wirksame Therapeutika bei der Behandlung der Psorias vulgaris erwiesen. Allerdings ist ihre Anwendung aufgrund der hypercalcämischen Nebenwirkungen eingeschränkt. Die Verbindungen führen zu einer Aktivierung der Proteinkinase C in Keratinozyten, so daß über diesen Weg eine Induktion der Differenzierung erreicht wird.

Erfindungsgemäß konnte nun gezeigt, werden, daß Calcitriol als auch Calcipotriol einen stärken aktivierenden Einfluss auf die Sphingosin-Kinase ausüben. Die Bildung von Sphingosin-1-phosphat führt zu einem antiproliferativen als auch differenzierungsfördernden Effekt in Keratinozyten.

Erfindungsgemäß kann daher in einer besonderen Ausführungsform dem Arzneimittel zur Behandlung von Hautkrankheiten, insbesondere von Psoriasis vulgaris, Acne vulgaris oder Dermatoheliosis Calcitriol oder Calcipotriol in üblichen Konzentrationen zugesetzt werden.

Die Erfindung betrifft daher auch die Herstellung eines Arzneimittels zur Behandlung von entzündlichen Hautkrankheiten, insbesondere von Psoriasis vulgaris, Acne vulgaris oder Dermatoheliosis. Das erfindungsgemäße Mittel enthält einen oder mehrere Wirkstoffe ausgewählt aus Sphingosin-1-Phosphat, Sphingosin-1-Phosphat-Derivaten der oben genannten Formel I und/oder Gemischen der vorgenannten Substanzen und/oder pharmazeutisch annehmbaren Salzen der vorgenannten Substanzen und einen pharmazeutisch annehmbaren Träger, Verdünner oder Exzipienten.
Als Trägersubstanzen kommen Phospholipide, wie Soja- und/oder Ei-Lecithine, natürliche und/oder semisynthetische Phospholipide in einer Konzentration von 0,1 bis 10 Gew.-%, bevorzugt 1 bis 5 Gew.-%, in Frage. Weiterhin kommen Glykosphingolipide in einer Konzentration von 0,01 bis 0,5 %, bevorzugt von 0,05 bis 0,2%,. in Betracht. Als weitere Lipide für den Arzneimittelträger können Phosphatidylcholin wie z.B. Phosphatidylcholin S100 (Hersteller: Lipoid KG, Ludwigshafen) oder auch Cholesterin verwendet werden. Vorzugsweise wird in dem Arzneimittel zur Behandlung von entzündlichen Hautkrankheiten Sphingosin-1-Phosphat verwendet. Sphingosin-1-Phosphat bzw. seine Derivat sollen im wesentlichen frei vom L-threo-Isomer sein. In einer besonders bevorzugten Ausführungsform ist das erfindungsgemäße Mittel in Liposomen verkapselt, ist topisch applizierbar und hat insbesondere die Form eines Hydrogels, Oleogels oder einer Lotion. D'er Wirkstoff, und. hier insbesondere Sphingosin-1-Phosphat, liegt im fertigen Präparat in Konzentrationen von 0,002 bis 0,05 mg/g vor. Besonders bevorzugt liegt eine Wirkstoffkonzentration im fertigen Präparat von 0,005 - 0,01 mg/g vor.

Zur Verkapselung der erfindungsgemäßen Wirkstoffe, d.h. von Sphingosin-1-Phosphat oder Sphingosin-1-Phosphat-Derivaten oder Gemischen davon, können die Verfahren angewendet werden, die dem Fachmann an sich wohl .bekannt sind. So kann man beispielsweise diese Wirkstoffe und die Liposomen-bildenden Substanzen in einem organischen Lösungsmittel lösen, die Lösung mit einer wäßrigen Phase mischen und gegebenenfalls nach Homogenisation das Lösungsmittel destillativ entfernen. Üblicherweise werden die 100- bis 500.000-fache : Gewichtsmenge Liposomen-bildende Substanz oder Substanzgemisch, bezogen auf den Wirkstoff, z.B. Sphingosin-1-Phosphat, verwendet.

Zur. Herstellung von Liposomen-Suspensionen verwendet man üblicherweise 0,5 bis 10 Gew.-% Phospolipid oder Phospholipid-Gemisch bezogen auf die wäßrige Phase. Geeignete Gemische können bis zu 60 Gew.-% Cholesterin und bis zu 30 Gew.-% Ladungsträger enthalten. Als Lösungsmittel verwendet man vorzugsweise Alkohole.

Da die Lipide oxidationsempfindlich sind, können auch Antioxidantien usw. zugesetzt werden.

Die Verkapselung von Sphingosin bzw. Sphingosin-1-Phosphat in Liposomen kann unter den gleichen Bedingungen durchgeführt werden, wie sie in vorbekannten Verfahren dieser Art beschrieben ist (siehe z.B. EP 0 605 497 oder DE 44 40 337).

Die optimale Wirkstoffkonzentration in den fertigen pharmazeutischen Präparaten ist naturgemäß von der Art des Wirkstoffes und der galenischen Zubereitung abhängig und muß im Einzelfall mittels der üblichen Vorversuche ermittelt werden. In der Regel wird es ausreichend sein, wenn man pharmazeutische Präparate anwendet, die 0,002 bis 0,05 mg und vorzugsweise 0,005 bis 0,01 mg Sphingosin, Sphingosin-1-Phosphat pro Gramm Präparat enthalten.

Die nachfolgenden Beispiele und Ergebnisse dienen zur näheren Erläuterung der Erfindung.

### 1. Beeinflussung der Keratinozyten-Proliferation

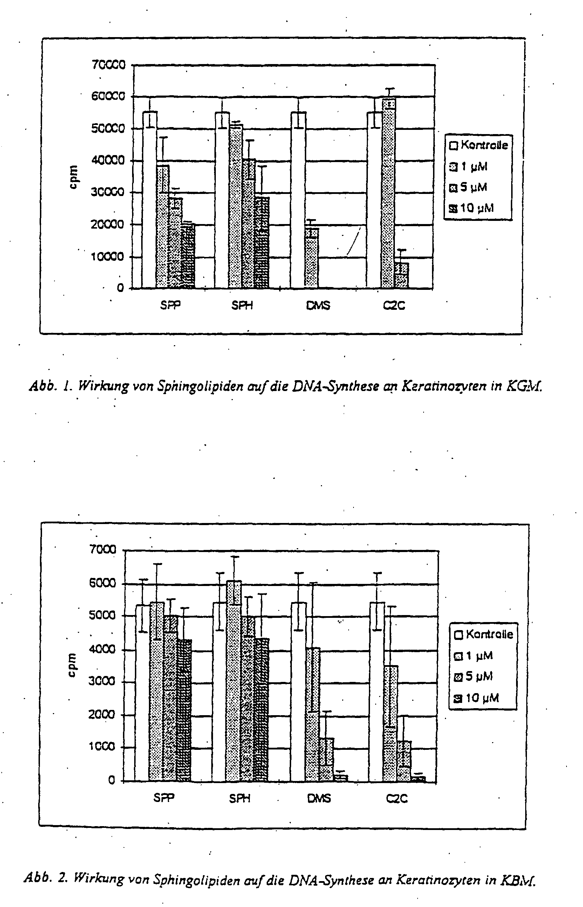

Die Proliferation von Keratinozyten wurde in Gegenwart von 1-10 *µ*M von Sphingolipid-Derivaten untersucht. Nach 4-tägiger Behandlung mit verschiedenen Sphingolipiden wurde in der logarithmischen Wachstumsphase der Keratinozyten der [³H]-Thymidineinbau in die DNA gemessen. Sphingosin-1-Phosphat (SPP) wirkte in KGM (keratinocyte growth medium) stark proliferationshemmend, dabei konnte eine ' ausgeprägte Konzentrationsabhängigkeit beobachtet werden. Eine Proliferationshemmung tritt bereits bei einer Konzentration von 1 *µ*M ein, 10 *µ*M SPP reduzierten den Thymidineinbau um ca. 63,1 % (Abb. 1). Damit es ebenso potent wie Calcipotriol D₃, welches bei Psoriasis vulgaris Verwendung findet. Sphingosin (SPH) zeigte ebenfalls eine wachstumshemmende Wirkung, aber der Effekt war schwächer ausgeprägt als bei SPP. Anhand des MTT-Tests wurde die Zellvitalität in Gegenwart von 1-25 *µ*M SPP untersucht, bis 10 *µ*M wirkten die Substanzen nicht toxisch. Andere Sphingolipid-Derivate wie Dimethylsphingosin (DMS) und C2-Ceramid (C2C) hemmten zwar ebenso den Thymidineinbau, diese Wirkung war jedoch allein auf die Toxizität beider Substanzen zurückzuführen. Dies zeigten mikroskopische Untersuchungen, der MTT-Test als auch durchflußzytometrische Untersuchungen zu Apoptose und Nekrose.

Die Versuche wurden auch in "keratinocyte basal medium" (KBM), ohne Supplemente), an kultivierten Keratinozyten durchgeführt. Damit konnte festgestellt werden, daß SPP bzw. SPH keine mitogenen Effekte an Keratinozyten besitzen (Abb. 2). DMS und C2C wirkten auch in diesem Medium toxisch.

### 2. Untersuchungen zur Fibroblasten-Proliferation

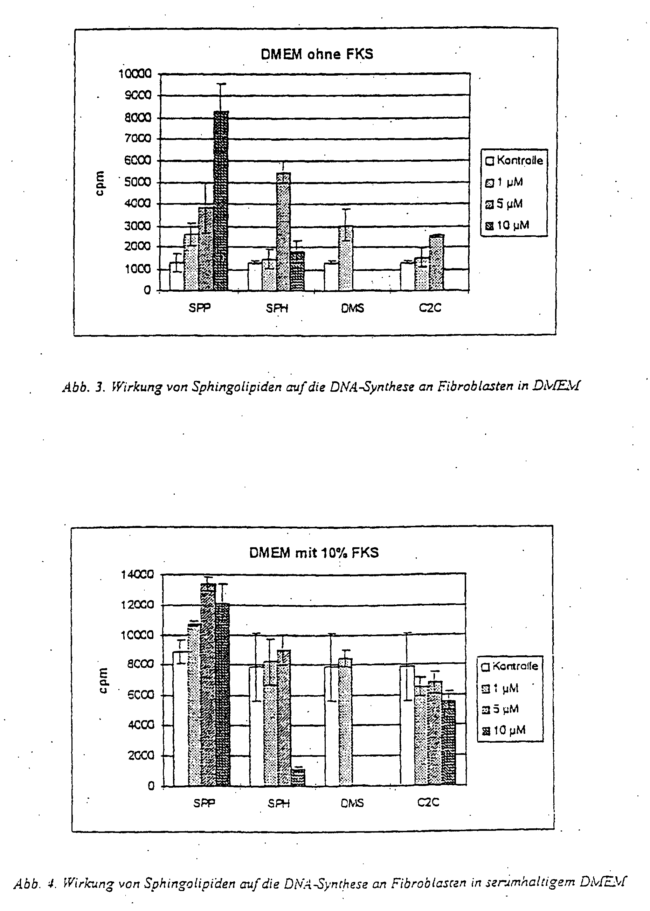

Das Wachstumsverhalten menschlicher. Fibroblasten in Gegenwart der Testsubstanzen wurde an nicht stimulierten Zellen verfolgt, die in serumfreiem Medium (nach Dulbecco modifiziertes Earle-Medium (DMEM) ohne fetales Kälber-Serum (FKS)) kultiviert wurden. Die Proliferation wurde durch SPH und insbesondere SPP konzentrationsabhängig stark' stimuliert (Abb. 3). Die höchste Aktivität von SPP wurde in der Konzentration von 10 *µ*M gemessen (ca. 6,5fach), aber auch 1 *µ*M bewirkten bereits eine wahrnehmbare Proliferationszunahme. Damit wird die proliferative Wirkung an Fibroblasten in den gleichen Konzentrationen hervorgerufen wie der antiproliferative Effekt an Keratinozyten. Auch SPH verstärkte bei Fibroblasten die Teilungsaktivität, wieder lag die Wirkstärke niedriger als bei SPP. Konzentrationen von 10 *µ*M SPH induzierten schon toxische Effekte. DMS und C2C zeigten in niedrigen Konzentrationen eine sehr geringe proliferationsfördernde Wirkung, Konzentrationen von 5 *µ*M und mehr wiesen eine ausgeprägte Toxizität auf.

Analoge Versuche wurden mit Zellen durchgeführt, die in serumhaltigem Medium kultiviert wurden und sich in der Wachstumsphase befanden. Wie zu erwarten, fielen die proproliferativen Effekte aufgrund der Überlagerung der Effekte von
Serumbestandteilen wesentlich geringer aus, ein antiproliferativer Effekt war jedoch nicht feststellbar (Abb. 4).

### 3. Einfluß auf die Differenzierung von Keratinozyten

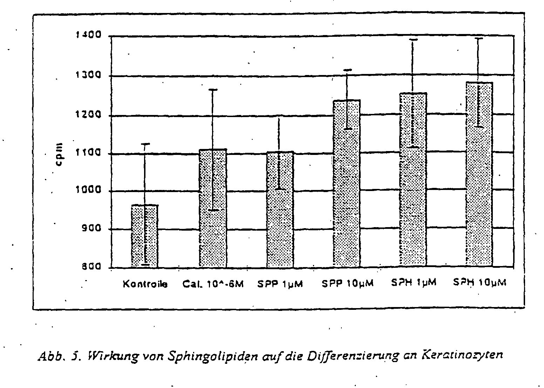

Nachdem gezeigt worden war, daß SPP die Proliferation von Keratinozyten hemmt, ohne daß damit eine vermehrte Toxizität verbunden ist, wurde der Differenzierungsgrad der Zellen nach Einwirkung der Sphingolipid-Derivate untersucht. Die Bestimmung der Transglutaminase-Aktivität zeigte, daß lediglich SPP und SPH die Differenzierung von Keratinozyten förderten - also genau die Lipide, die proliferationshemmend wirken. Als Vergleich wurde der aktive Metabolit von Vitamin D₃ herangezogen, der ebenfalls eine Differenzierung fördert. Die Zunahme der Differenzierung von Keratinozyten durch SPP und SPH ist demnach durchaus vergleichbar mit der Wirkung von Calcitriol D₃ (Abb. 5).

### Beispiel 1

### Herstellung von Sphingosin-1-Phosphat enthaltenden Liposomen-Suspensionen

Im folgenden wird die Herstellung von Liposomen-Suspensionen mit Hilfe der Filmmethode mit anschließender Hochdruck-Homogenisation beschrieben.

7,5 g Phosphatidylcholin S100 (Hersteller: Lipoid KG, Ludwigshafen), 0,75 g Cholesterin und 15 mg Sphingosin-1-Phosphat (D-Erythro-Form) werden in 5 ml 96%igem Ethanol gelöst. Dann wird die Lösung schonend mittels eines Rotationsverdampfers zur Trockne eingeengt, wobei sich ein Lipidfilm an der Kolbenwand bildet. Der Lipidfilm wird mit 141,7 g destilliertem Wasser abgelöst. Anschließend wird die erhaltene Liposomen-Suspension (MLV) mit einem Hochdruck-Homogenisator bei 950 bar homogenisiert und durch einen Filter von 0,2 *µ*m filtriert. Die resultierende Liposomen-Suspension enthält Liposomen mit einer mittleren Größe von 35 nm. Der Gehalt an Phosphatidylcholin liegt bei 48 mg/g; der Gehalt an eingebautem Wirkstoff beträgt 0,1 mg/g.

### Herstellung eines liposomalen Lipogels

Die gemäß oben genanntem Beispiel 1 hergestellten Liposomen werden 1:10 (G/G) mit Citrat-Phosphatpuffer pH 5,5 verdünnt. Als Gelbildner wird Ketrol TF® aufgestreut und dispergiert. Es wird schwach opaleszentes Gel erhalten, welches noch konserviert wird. Die Wirkstoffkonzentration liegt bei 10 µg/g.

## Patentansprüche

1. Verwendung von Sphingosin-1-Phosphat und Sphingosin-1-Phosphat-Derivaten der folgenden Formel I. 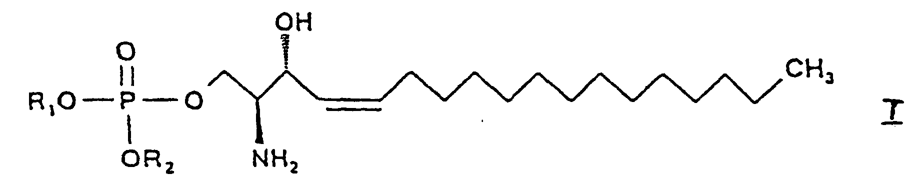 worin R₁ ein AIkvlrest mit 1 bis 6 C-Atomen
und R₂ = Wasserstoff oder 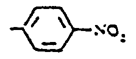 ist, und/oder pharmazeutisch annehmbaren Salzen der vorgenannten Substanzen, und/oder Gemischen der vorgenannten Substanzen zur Herstellung eines Arzneimittels zur Behandlung von entzündlichen Hautkrankheiten.

2. Verwendung gemäß Anspruch 1, dadurch gekennzeichent. daß die entzündlichen Hautkrankheiten Psoriasis vulgaris, Acne vulgaris oder Dermatoheliosis sind.

3. Verwendung gemäß Anspruch 1 oder Anspruch 2, **dadurch gekennzeichnet, daß** das Sphingosin-1-Phosphat oder die Sphingosin-1-Phosphat-Derivate im wesentlichen kein L-threo-Isomer enthalten.

4. Verwendung gemäß einem der vorhergehenden Ansprüche 1 bis 3, **dadurch gekennzeichnet, daß** das Arzneimittel einen pharmazeutisch annehmbaren Träger. Verdünner oder Exzipienten enthält.

5. Verwendung gemäß Anspruch 4, **dadurch gekennzeichnet, daß** das Arzneimittel als Verdünner Sphingosin enthält.

6. Verwendung gemäß Anspruch 4 oder 5, **dadurch gekennzeichnet, daß** das Arzneirmittel in Liposomen verkapseltes Sphingosin-1-Phosphat und Sphingosin-1-Phosphat-Derivate und/oder Gemische davon enthält.

7. Verwendung gemäß Anspruch 4, 5 oder 6, **dadurch gekennzeichnet, daß** das Arzneimittel topisch applizierbar ist und in Form eines Hydrogels. Oleogels oder einer Lotion vorliegt.

8. Verwendung gemäß einem der vorhergehenden Ansprüche 1 bis 7, **dadurch gekennzeichnet, daß** das Arzneimittel eine Wirkstoffkonzentration. insbesondere von Sphingosin-1-Phosphat, im fertigen Präparat von 0,002 bis 0,05 mg. insbesondere von 0.005 bis 0,01 mg/g. enthält.

## Claims

1. Use of sphingosine 1-phosphate and sphingosine 1-phosphate derivatives of the following formula I, 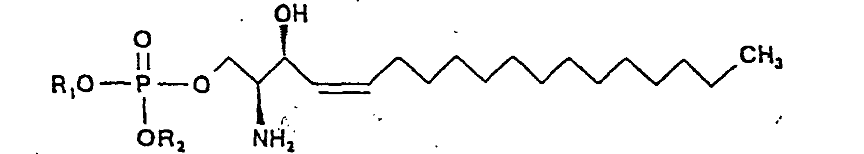 where R1 is an alkyl residue with 1 to 6 C atoms
and R2 is hydrogen or 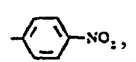 and/or of pharmaceutically-acceptable salts of the afore-mentioned substances, and/or mixtures of the afore-mentioned substances for the preparation of a drug for the treatment of inflammatory dermatoses.

2. Use according to claim 1, **characterized in that** the inflammatory dermatoses are *psoriasis vulgaris, acne vulgaris* or dermatoheliosis.

3. Use according to claim 1 or 2, **characterized in that** the sphingosine 1-phosphate or the sphingosine 1-phosphate derivatives contain essentially no L*-threo* isomer.

4. Use according to one of the preceding claims 1 through 3, **characterized in that** the drug contains a pharmaceutically-acceptable support, diluent or excipient.

5. Use according to claim 4, **characterized in that** the drug contains sphingosine as a diluent.

6. Use according to claim 4 or 5, **characterized in that** the drug contains sphingosine 1-phosphate and sphingosine 1-phosphate derivatives and/or mixtures thereof encapsulated in liposomes.

7. Use according to claims 4, 5 or 6, **characterized in that** the drug can be applied topically, and is present in the form of a hydrogel, oleogel, or lotion.

8. Use according to one of the preceding claims 1 to 7, **characterized in that** the drug contains a concentration of the active substance, particularly of sphingosine 1-phosphate, of 0,002 to 0,05 mg/g and particularly of 0,005 to 0,01 mg/g in the finished preparation.

## Revendications

1. Utilisation de la sphingosine-1-phosphate et de dérivés de la sphingosine-1-phosphate de la formule I suivante : 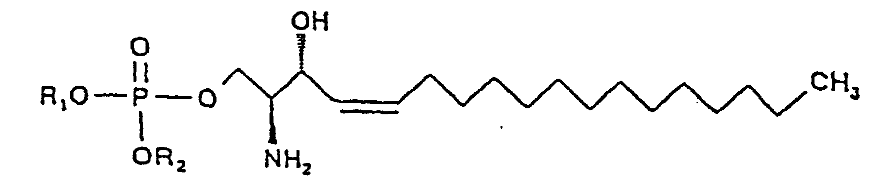 dans laquelle R₁ est un résidu alkyle avec 1 à 6 atomes de carbone
et R₂ est un hydrogène ou 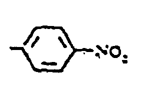 et / ou de sels acceptables sur le plan pharmaceutique des substances susmentionnées et / ou de mélanges des substances susmentionnées pour préparer un médicament pour traiter des maladies cutanées inflammatoires.

2. Utilisation selon la revendication 1, **caractérisée en ce que** la « maladie cutanée inflammatoire » est le psoriasis vulgaire, l'acné vulgaire ou la dermatohéliose.

3. Utilisation selon la revendication 1 ou la revendication 2, **caractérisée en ce que** la sphingosine-1-phosphate ou le dérivé de la sphingosine-1-phosphate, est essentiellement sous la forme de l'isomère L-thréo.

4. Utilisation selon d'une des revendications précédentes 1 à 3, **caractérisée en ce que** le médicament contient un vecteur, un diluant ou un excipient acceptable sur le plan pharmaceutique.

5. Utilisation selon la revendication 4, **caractérisée en ce que** le médicament contient de la sphingosine en tant que diluant.

6. Utilisation selon la revendication 4 ou la revendication 5, **caractérisée en ce que** le médicament contient la sphingosine-1-phosphate, le dérivé de la sphingosine-1-phosphate ou leur mélange à l'état encapsulé dans des liposomes.

7. Utilisation selon la revendication 4, 5 ou 6, **caractérisée en ce que** le médicament peut être appliqué topiquement et se présente sous la forme d'un hydrogel, d'un oléogel ou d'une lotion.

8. Utilisation selon l'une des revendications précédentes 1 à 7, **caractérisée en ce que** le médicament a une concentration en substance active et, en particulier, en sphingosine-1-phosphate dans la préparation prête à l'emploi de 0,002 à 0,05 mg / g et surtout de 0,005 à 0,01 mg / g.
